# EUROPEAN PATENT APPLICATION

(11) **EP 2 478 779 A1**
(43) Date of publication of application: **25.07.2012**
(21) Application number: 11151899.9
(22) Date of filing: 24.01.2011
(51) Int. Cl.: A23L 1/29, A23L 1/302, A23L 1/303, A23L 1/308, A23L 1/03, A61K 31/702, A61K 31/715, A61K 31/716, A61K 31/717, A61K 38/47

(54) **Composition for providing beneficial health effects**

(71) Applicant: Biotechnobel, 1190 Brussel (BE)
(72) Inventor: Bathaei, Saeid, 1190 Brussel (BE)
(74) Representative: Duyver, Jurgen Martha Herman

(57) **Abstract**

The composition is based on the active role of certain dietary fibres as functional food ingredients in combination with a blend of enzymes which optimize the functioning of the gut flora and in addition with a supporting blend of nutrients in order to help the restoration of the colonic epithelium.

## Description

The present invention relates to a composition for providing beneficial health effects.

The composition is based on the active role of certain dietary fibres as functional food ingredients in combination with a blend of enzymes which optimize the functioning of the gut flora and in addition with a supporting blend of nutrients in order to help the restoration of the colonic epithelium (colonocytes).

Dietary fibres are an important part of a healthy and balanced nutrition and its role in the digestion and metabolism of food is more and more being investigated, although some aspects of its biological effects are not yet fully understood. Food scientists however agree that dietary fibres are beneficial for human health and form an essential part of any well balanced diet.

Dietary fibres are generally regarded as the indigestible portion of plant or vegetable foods. They are carbohydrates, more specifically non-starch poly- and oligosaccharides, and lignin, primarily derived from plant cell walls that cannot be hydrolyzed by human digestive enzymes. However, they often can be fermented by intestinal bacteria to produce hydrogen, methane, carbon dioxide, water and short chain fatty acids (SCFA).

Classification of dietary fibres can be done based on different parameters. They can for example be classified based on their fermentablility or viscosity but generally they are classified based on their solubility. Hereby, the dietary fibres are divided into two different categories, namely insoluble and soluble dietary fibres. Both types have different structures and functions and are beneficial to the human digestion.

Insoluble fibres are metabolically inert, but act by absorbing water throughout the digestive system, hereby easing defecation. Primary benefits of these types of fibres are thus that they facilitate regularity and alleviate constipation.

The soluble fibres are those fibres which can be fermented in the large intestine, more specifically in the colon. These are also the fibres which are digested by endogenous bacteria into gases and physiologically active by-products.

In the large intestine, the fibres primarily serve as a substrate for micro-organisms of the intestine. The epithelium of the large intestine, of which its importance in human health is more and more being acknowledged, is an ideal environment for endogenous micro-organisms. Besides from direct access to food, the epithelium offers a supportive base to the endogenous bacteria to which they can adhere optimally.

Dietary soluble fibres in the large intestine, which can not be hydrolyzed by human enzymes, are hydrolysed by gut bacterial enzymes and fermented by these endogenous bacteria. By doing this, these bacteria will produce hydrogen, methane, short chain fatty acids, etc. Especially the short chain fatty acids are very beneficial for the human health and thus form one of the most important benefits of dietary fibres.

Short chain fatty acids (SCFA) are by-products of the fermentation of soluble fibres in the colon. SCFA are involved in numerous physiological processes promoting health. Amongst others they stabilize blood glucose levels by acting on pancreatic insulin release and liver control of glycogen. They provide nourishment of colonocytes, particularly by the SCRA butyrate, they reduce blood levels of LDL cholesterol and triglycerides responsible for atherosclerosis, they lower colonic pH, stimulate the production of T helper cells, antibodies, etc. and improve barrier properties of the colonic mucosal layer.

The production of SCFA is thus one of the main reasons why dietary fibres are so beneficial for our health.

An important class of the soluble fibres are the oligosaccharides. They are formed by short to medium length sugar chains. When oligosaccharide fibres are consumed the undigested portion serves as food for the gut flora. Depending on the type of oligosaccharide, different bacterial groups are stimulated or suppressed. Several clinical studies have shown that administering oligosaccharides can increase the number of friendly or beneficial micro-organisms while reducing the population of harmful micro-organisms bacteria.

Fructo-oligo saccharides (FOS) are mainly found in vegetables and consist of short chains of fructose molecules. Inuline, another dietary fibre has approximately the same structure as FOS and also consists of fructose molecules. It is however far more polymerized and is therefore considered to be a polysaccharide.

Other oligosaccharide fibres are xylo-oligo saccharids (XOS) which consist of respectively mannose and xylose units. Xylo-oligo saccharids are beneficial to human health for several reasons. For example, they support the selective growth of the beneficial bacteria Bifidobacterium spp and bacteroides, which have important biological effects due to the production of SCFA and they facilitate the absorption of nutrients. Xylo-oligo saccharids are considered to be advantageous over other non-digestible oligosaccharides in terms of health. They naturally occur in fruits, vegetables, bamboo, honey and milk and can be produced on an industrial scale from xylan-rich materials such as rice bran.

Dextrins are gluco-oligo saccharids (GOS), a linear complex of oligo saccharids that cannot be hydrolysed by the amylolytic enzymes, and hence are considered as fibres with potential positive effects for the gut flora.

Several dietary fibres, such as the oligosaccharides discussed have also been defined as prebiotics. For a food ingredient to be classified as a prebiotic it must fulfil the following criteria:
i) neither be hydrolysed, nor absorbed in the upper gastrointestinal tract,
ii) be selectively fermented by one or a limited number of potentially beneficial gut bacteria commensal to the colon, and
iii) be able to alter the gut flora towards a healthier composition, by increasing friendly bacteria and reducing harmful gut flora.

Since dietary fibres provide such a broad range of nutritional and health benefits, it is not a surprise that they are added to many food products and are provided as separate food supplements or prebiotic compositions.

WO 2007/050656 A2 for example describes a food supplement based on dietary fibres. The food supplement is a fibre formulation comprising in its primary embodiment partially-hydrolyzed guar gum (PHGG) and fructo-oligo saccharids (FOS), wherein the dietary fibre formulation exhibits a prebiotic potential greater than a prebiotic potential of PHGG and FOS individually.

WO 2004/052121 describes prebiotic compositions comprising soluble fibres, in particular fructo-oligo saccharids (FOS) and galacto-oligo saccharids and their use in the treatment of prevention of gastrointestinal tract disorders, such as inflammatory bowel disease (IBD) diarrhoea and constipation.

US2008/0261919A1 describes synergistic compositions comprising prebiotic components selected from a large group comprising amongst many others modified or unmodified starch and partial hydrolysates thereof, fructo-oligo saccharids, trans-galacto-oligo saccharids, xlylo-oligo saccharids (XOS), betaglycan and partial hydrolysates thereof, end if desired other plant extracts, mineral components, vitamins and additives.

All of the previously described compositions relate to prebiotic mixtures of dietary fibres and provide some sort of beneficial health effects by stimulating the micro-organisms predominantly present in the large intestine. However, none of the compositions manages to stimulate the micro bacterial gut flora in an optimal manner.

WO 2007/050656 A2 describes a synergistic effect when mixing partially-hydrolyzed guar gum (PHGG) and fructo-oligo saccharids (FOS). This effect is however small, and although the mixture is selectively fermented and does stimulate some beneficial gut bacteria, this effect is not equal divided over all beneficial bacteria. Some bacteria will be strongly stimulated while others, for example beneficial bacteria capable of only slowly metabolizing FOS or PHGG, will be overgrown by the strongly stimulated bacteria. Therefore, the beneficial microbial flora will not be very diverse and will only comprise a few species.

WO 2004/052121 describes a mixture of FOS and galacto-oligo saccharids and their beneficial effects, as well as the synergy between these two prebiotic fibres. In other preferred embodiments, also other prebiotic oligosaccharides such as XOS are comprised in the composition.

As mentioned before US2008/0261919A1 describes a composition comprising a wide variety of dietary fibres.

These two last publications thus describe compositions which comprise more prebiotic compounds, and will thus provide a more varied nutrition for beneficial microbial bacteria.

However, since the metabolic rates by which the different bacteria break down these different prebiotic fibres, still varies depending on several factors, such as species, the growth of these different bacteria will still not be equally stimulated. As of result of this, there will be a lack of diversity in the beneficial microbial flora and beneficial health such as an improved food digestion etc. will be limited.

There is thus a need for a composition which stimulates a broader range of beneficial micro-organisms and more equally stimulates these various species.

It is therefore a goal of the present invention to provide such a composition which offers an overall broader prebiotic activity, and stimulates the beneficial gut flora more equally and in a more optimal manner.

Thereto, the composition comprises dietary fibres as functional food ingredients in combination with a blend of enzymes which optimize the functioning of the gut flora and in addition with a supporting blend of nutrients in order to help the restoration of the colonic epithelium.

Thereto, more than one type of dietary fibres is used. More specifically more than one type of oligosaccharides is used in the composition to restore the equilibrium of the gut flora. The different kinds of dietary fibres are present in the composition according to the present invention, allow to stimulate a higher number and higher range of species of the beneficial gut flora. This makes it possible to preserve the biodiversity of, gut flora.

According to the present invention, the dietary fibres comprise a mixture of xylo-oligo saccharids (XOS) and/or xylane, fructo-oligo saccharids (FOS), and gluco-oligo saccharids (GOS).

These dietary fibres comprise a broad and varied mixture that will stimulate a diverse group of beneficial bacteria such as several species of bifidobacteria and/or lactobacillus. Inuline is a polymer which will be cleaved by the bacterial enzymes to FOS and will have a similar prebiotic activity. Although the number of different types of dietary fibres is still rather limited in this composition, these specific types of fibres offer very beneficial effects for the gut flora, and a wide variety of different beneficial micro-organisms will be stimulated.

Xylo-oligo saccharids provide an additional beneficial effect. Xylo-oligo saccharids are considered advantageous over other non-digestible oligosaccharides in terms of both health and technological related properties. Rice bran is riche in Xylans. Xylans are chemically complex, and their degradation requires multiple enzymes. The expression of these enzymes by the bacteria of the intestine varies considerably from one species to another. Polysaccharides of cellular wall of plants like rice bran (insoluble fiber) are not hydrolysed in the stomach and the small intestine. After reaching the colon, these materials are used as source of fermentable carbohydrate available for the endogenous bacteria. The species of bacteroides constitute an important part of the bacterial population of the colon. In the human colon, bacteroides are major xylanolytic bacteria. They can ferment xylans and use xylans as primary source of carbohydrate. They can grow on xylans and produce xylo-oligo saccharids (XOS).

It has been found that the xylo-oligo saccharids selectively support the growth of the micro organisms in the human colon in particular Bacteroides and prevent the production of acid by Streptococcus. In addition, supplements or XOS and FOS augment significantly growth of the bifidobacteria of lactobacillus in the cecum. Additionally, XOS and FOS have a positive influence on metabolic anomalies related to the diabetes.

Dextrins are gluco-oligo saccharids (GOS), a linear complex of gluco-oligosides (of the oligoside of glucose) that cannot be hydrolysed by the amylolytic enzymes. The shortest of dextrins is isomaltose, made up of 2 units of glucose.

The gluco-oligo saccharids are made up mainly of glucose. The hydrolysis of starch produces gluco-oligosides.

From the point of view of digestibility, gluco-oligo saccharids have a particular alfa(1-2) link at the nonreducing end of the oligosaccharide which makes it possible to block the hydrolytic action by salivary and intestinal digestive enzymes.

Consequently they reach the colon intact. The gluco-oligo saccharids (GOS) belong to the group of prebiotics which stimulate the growth and/or the activity of the salutary bacteria in the colon.

Balanced gut flora plays an important part in the natural defense of the human body. The gluco-oligo saccharids support the natural defense of the body via the gut flora and indirectly inhibits the connection of Escherichia coli, salmonella typhimurium and Clostridium to the colon. Thus reduces the risk of infection and interacts directly with the immunity cells which are potentially able to prevent allergies and reduce infectious diseases.

Constipation is a frequent problem, in particular among the elderly and the pregnant women. For these individuals the consumption of gluco-oligo saccharids can offer a relief to their complaints.

Gluco-oligo saccharide is an oligosaccharide which is more easily consumed by bacteroides and bifidobacteries than by clostridium. Even if these 3 families are an important combination in an average individual, Clostridium cannot be regarded as a family favourable to intestinal equilibrium.

These elements confirm that gluco-oligo saccharids are a good substrate to the populations of Bacteroides, and that it's consumption supports the growth of those elements which are the adverse to other prebiotic compositions which stimulate preferably bifidobacteries.

Preferably, the mixture comprises 20% wt. FOS, 30% wt. GOS and 50% wt. with respect to the weight of the mixture. It has been found that such a mixture allows an especially good stimulation of a broad range of beneficial micro-organisms in a more equal way. Although these values are especially preferred it has been found that similar effects are observed within the following ranges: 15 - 25% wt. FOS, 25 - 35% wt. GOS and 45 - 55% wt. with respect to the weight of the mixture, always keeping in mind that when making combinations of different values for these ranges the sum of the different wt.% is 100wt.%.

The composition according to the present invention not only comprises the dietary fibres which have a prebiotic beneficial health effect. It also comprises at least one enzyme capable of breaking down these dietary fibres.

In this way the enzyme will cleave the fibres, and split these fibres into smaller fibre fragments, mainly oligosaccharides, possibly also in di- and monosaccharides which can be fermented more easily and faster by the different species of beneficial bacteria. In case of inflammatory bowel disease (IBD), these enzymes are not produced anymore by the gut bacteria and hence help is needed. Therefore, the composition according to the present invention will offer a broadly applicable prebiotic which will activate the beneficial microbial population in a optimal manner and offer them a selective growth advantage in comparison to non beneficial gut bacteria, such as coliforms, Clostridum, sulphate reducing Bacteria, etc.

The composition according to the present invention will have a further advantage over compositions containing partially hydrolysed fibres, thus fibres which are already cleaved, such as those described in US2008/0261919A1. Because such enzymes in the composition according to the present invention generally have a slow onset when they enter our gastrointestinal tract, the hydrolyzation will occur mainly in the large intestine. Therefore, only a minor extent of hydrolysed fibres will be digested by the micro-organisms in the small intestine. This is in contrast with compositions comprising fibres which have already been hydrolysed outside of our body such as US2008/0261919A1. In these compositions, the fibres enter our gastro-intestinal tract in a hydrolyzed and cleaved state, resulting in a massive digestion in the small intestine such that they can no longer turn into gel. The composition according to the present invention will thus have a more effective prebiotic action compared to compositions containing hydrolyzed fibres.

In a preferred embodiment of the present invention the enzyme is capable of digesting down those types of dietary fibres which are more difficult to metabolize by the endogenous beneficial intestinal bacteria than other dietary fibres.

As such the enzyme will stimulate the growth of the bacteria which predominantly feed on those fibres which are more difficult to metabolize and which therefore, often grow slower. As such, these micro-organisms will be able to grow faster and will be more capable of competing with the other beneficial bacteria. Therefore, a more diverse beneficial gut flora will arise, of which many of the known beneficial health effects will be further improved.

An increased diversity in the gut flora is a serious advantage and obtaining it is an important subject of this invention. Stimulation of only one microbial species, such often occurs with the traditional food supplements, can cause a domination of this one species in the gut flora and a suppression of other species and reduce biodiversity of the flora, which can further cause a number of diseases of the intestines such as for example several metabolic diseases.

Since micro-organisms predominantly feeding on those fibres which are difficult to metabolize use different substrates, they often have a specific metabolism and thus often also produce other important by-products such as specific SCFAs which are only to a minor extent produced by the other beneficial bacteria. A more diverse beneficial bacterial population will thus offer a broader beneficial activity which will have a very positively influence on both digestion as on health in general.

In a further preferred embodiment of the present invention, Xylanase is added as enzyme.

The human body does not produce xylanase. This belongs to the family of the enzymes of glucanase which are characterized by their capacity to break up various xylans to produce the xylo-oligo saccharids with short chain lide xylose. This enzyme is important for a smooth digestion of fibres. The xylanase liquidates polysaccarides on the wall of vegetable cells in particular the grains. Scientific studies on animals show that xylanase reduces the population of certain pathogenic bacteria particularly the salmonella.

In the human colon, bacteroides are major xylanolytic bacteria. They can ferment xylans and use them as a primary source of carbohydrate.

Although xylanes and xylo-oligo saccharids XOS both provide substantial health benefits, as is explained here above, and they are very important food sources for some important beneficial bacteria, they are still difficult to metabolize by these bacteria, or their metabolism is quite slow, causing these bacteria to have a selective disadvantage compared to beneficial bacteria predominantly feeding on other types of dietary fibres. Especially Bacteroides species, such as Bacteroides fragilis, bacteroides Eggerthii and Bacteroides ovatus, which predominantly feed on xylan, have it difficult to compete with other micro-organisms in terms of growth, etc. Therefore, the enzyme which is added to the composition is preferably xylanase. Xylanase will further break down the XOS and the xylanes, giving the bacteria that predominantly feed on these fibres, the opportunity to metabolize and grow faster and compete with the other beneficial bacteria.

Therefore, these beneficial bacteria will be more strongly stimulated with the composition according to the present invention, than with the compositions of the prior art, providing a more diverse and healthier gut flora.

Pectin is another polysaccharide dietary fibre with a complex structure composed of amongst others galacturonic acid. However it is preferred not to have pectine as a substrate for the bacterial flora in the colon in case of inflammatory bowel disease. In a more preferred embodiment this is prevented by adding pectinase to the composition for increasing the digestion of pectin in the small intestine.

Pectinase is a generic term for the enzymes which are able to hydrolyse pectin. The pectinase enzymes are exopolygalacturonases, pectinase, methylesterases and lyases. Pectinases hydrolyses pectin. They reduce the number of toxic metabolites, such as ethanol, produced during fermentation by pectin.

In a further preferred embodiment of the present invention, the composition comprises additional enzymes to improve digestion in the small intestine.

These additional enzymes do not digest the fibres comprised in the composition according to the present invention. They will rather help digest nutrients such as starch, fats or some sugars such as lactose in the small intestine. Preferably all digestible nutrients comprised in food taken up by the human body are digested in the small intestine, so that only the indigestible portion of the food (including dietary fibres) is passed on to the large intestine. If a substantial portion of the digestible nutrients is passed on the large intestine, several health problems and diseases might arise. The additional enzymes of the composition according to the present invention are preferably identical or functionally similar to enzymes occurring naturally in the small intestine. As such, these additional enzymes provide a supplement to the natural enzymes, and thus increase the capacity of the small intestine to digest nutrients.

The advantages of adding these additional enzymes to the composition are an improved digestion in the small intestine and a decreased amount of non-digestible nutrients in the large intestine. As of result of the latter, there will be a decrease in non beneficial gut bacteria in the large intestine which are possibly feeding on these digestible nutrients. Furthermore, stool problems related to a high the amount of starch in the large intestine will be eliminated. The addition of these additional enzymes will thus provide an overall better digestion and a healthier gastro-intestinal tract.

The additional enzymes are preferably chosen from the group of lactase, pectinase, amylase, amyloglucosidase, lipase, xylanase and combinations of two or more thereof.

More preferably all of the above mentioned enzymes are comprised in the composition according to the present invention.

In a preferred embodiment of the present invention, the composition is provided in a substantially dry formulation.

In a dry formulation, the composition will be preserved longer, and the enzymes comprised in the composition will not be in an inactive state. This will eliminate the risk that the enzymes will start digesting and cleaving the fibres before the composition enters the human gastrointestinal tract.

The composition is thus preferably provided in a formulation such as a powder, possibly compressed in e.g. a tablet or pill or encapsulated in e.g. a gelatine capsule. As such, the different components will be in a dry matrix, unable to interact with each other.

The composition is further protected against premature enzymatic degradation because the enzymes comprised therein are generally heat sensitive. As such, their optimal temperature is 37°C, or human body temperature. The risk that the composition according to the present invention is prematurely degraded when stored in a dry environment at room temperature is thus minimal.

The composition comprises at least one nutrient which is directly supporting colonic epithelium cells.

Preferably the nutrients are chosen from the group consisting of glutamine, vitamin A, vitamin C, vitamin D, vitamin E, vitamin B6, vitamin B9, Zinc and combinations thereof.

For example glutamine helps to improve the functions of the intestinal wall by stimulating the proliferation of enterocytes and colonocytes. Friendly bacteria adhere better to healthy colonocytes improving the barrier function of the colon wall.

Vitamin A is also involved in the immune system and is as such able to prevent certain diseases. In combination with Zinc, and glutamine it will also reduce the intestinal permeability. Vitamin C on the other hand plays an important role in the immune system by acting as an antioxidant. As such, it is able to reduce chronic inflammation caused by oxidative stress.

The quantity of glutamine in the composition is further preferably 2-10 wt.%, preferably 5-10 wt.%, with respect to the total weight of the composition.

Glutamine is an important nutrient providing several additional beneficial health effects as described here above. Therefore, the weight percentage of glutamine is preferably higher than that of the other nutrients.

The quantity of enzymes in the composition is preferably 1-5 wt.%, preferably 2-3 wt.% with respect to the total weight of the composition.

In this context, enzymes indicate the total quantity of both enzymes digesting dietary fibres and additional enzymes. Since enzymes act as biocatalysts, a small quantity will be sufficient to provide necessary enzymatic activity in the intestines.

The quantity of vitamins and minerals in the composition is preferably 0,1-5 wt%, preferably 1-3 wt.%, with respect to the total weight of the composition.

Cofactors such as vitamins and zinc can already provide a sufficient beneficial health effect in such small quantities.

In further preferred embodiments of the present invention, beneficial health effects are chosen from the group consisting of improvement of food digestion, treatment of diarrhoea, treatment of constipation, treatment of irritable bowel syndrome, treatment of inflammatory bowel disease, treatment of crohn's disease, treatment of obesity, treatment of dyslipidemia, and combinations of two or more thereof.

The composition can be used for several different purposes, such as these which are described here above. The composition can be for example used for the improvement of digestion, but it has also proven to be quite beneficial in the treatment of more complex diseases such as crohn's disease, inflammatory bowel disease etc.

The invention is however not restricted to this group of beneficial health effects, and the person skilled in the art will be capable of determining which other health effect can also be stimulated, or which other diseases can also be treated by the composition according to the present invention.

The composition according to the present invention is preferably intended for administration in a dose corresponding to 5-20 g per day, preferably 5-10 g per day.

The person skilled in the art will be capable of determining the exact dose to be administered. In case the composition is administered as a food supplement for a healthy person, to improve digestion or provide an overall healthier intestinal flora, 5 g per day should for example suffice.

The composition is further preferably intended for daily administration.

When administered daily, the gut flora will be frequently stimulated and an almost continuous flow of the composition according to the present invention will be supplied to the gut flora. As such, a daily administration will provide optimal beneficial health effects.

The present invention also relates to the use of the composition described above, wherein the composition is administered in a dose corresponding to 5-20 g per day, preferably 5-10 g per day.

Preferably, the composition is administered daily.

The invention will be further elucidated by one example of a preferred embodiment of the composition given below.

The different components of the preferred compositions and their exact amounts will be given in table form.

### Example 1:

**Table 1: The ingredients of the preferred composition of example 1 expressed %weight with respect to the total weight of the composition.**

| **Ingredients** | **% wt.** |
|---|---|
| **Enzymes (Xylanase, pectinase, lactase, amylase, Amyloglucosidase)** | **2,12** |
| **Dietary fibers (FOS, GOS, XOS)** | **91** |
| FOS (fructo-oligosaccharides) | 17 |
| GOS (gluco-oligosaccharides) | 28,5 |
| XOS (xylo-oligosaccharides) | 45,5 |
| **Glutamine** | 5,6 |
| **Vitamins and minerals** | 1,3 |
| Vitamins (A,C,D,E, B6,B9) | 1,1 |
| Minerals (Zinc) | 0,18 |

The composition according to example 1 comprises FOS, GOS en XOS as dietary fibres and thus offers a broad and varied mixture that will stimulate a diverse group of beneficial bacteria such as several species of bifidobacteria, bacteroides and/or lactobacillus. These specific types of fibres offer very beneficial effects for the gut flora, and a wide variety of different beneficial micro-organisms will be stimulated.

Besides the synergistic prebiotic effects of FOS (fructo-oligo saccharids) and GOS (gluco-oligo saccarids), the xylo-oligo saccharids (XOS) provide an additional beneficial effect to this mixture. XOS are considered advantageous over other non-digestible oligosaccharides and will specifically favour the selective growth of Bifidobacterium spp. and species such as Bacteroides fragilis, Bacteroides Eggerthii and Bacteroides ovatus.

In total, these dietary fibres account for 91% wt. of the composition and thus provide for the vast majority of this mixture.

Xylanase is added as an enzyme which will help digest the XOS fibres in the large intestine, and as such predominantly stimulate the bacterial species having difficulties competing with other bacteria in the gut flora.

Other enzymes that are added to the composition of example 1 are pectinase, lactase, amylase and amyloglucosidase, lipase and xylanase.

Pectinase is another enzyme which digests dietary fibres, more specifically pectinase digests pectin, a dietary fibre frequently occurring in a normal nutritional pattern. As such, pectinase will improve the digestion of pectin in the large intestine, and will prevent the formation of toxins, and finally it will further stimulate the beneficial gut flora comprised therein.

Lactase, amylase and amyloglucosidase are additional enzymes added to the composition to improve the digestion in the small intestine. They reinforce the enzymes naturally occurring in the small intestine and improve the digestion of lactose and starch. Furthermore, they prevent lactose or starch from reaching the large intestine where they might cause health problems or diseases.

In total, all enzymes and additional enzymes comprised in the composition of example 1 account for 2,12% of the total composition.

5,6 wt.% of glutamine and 1,3 wt.% of vitamins (A,C,D,E, B6,B9) and minerals (Zinc) are added to the composition. These nutrients will stimulate the enterocyte and colonocyte proliferation.

The composition of example 1 is preferably administered daily in a dose of 5 g/day. If the composition is administered to people suffering from metabolic diseases, etc. the dose is preferably increased to 20-25 g/day.

## Claims

1. Composition for providing beneficial health effects based on the active role of certain dietary fibres as functional food ingredients in combination with a blend of enzymes which optimize the functioning of the gut flora and in addition with a supporting blend of nutrients in order to help the restoration of the colonic epithelium, wherein the dietary fibres comprise a mixture existing of xylo-oligo saccharids (XOS) and/or xylane, fructo-oligo saccharids (FOS) and gluco-oligo saccharids (GOS) and wherein at least one of the enzymes is capable of breaking down at least one type of these dietary fibres.

2. Composition as claimed in claim 1, **characterized in that** the mixture comprises 20% wt. FOS, 30% wt. GOS and 50% wt. with respect to the total weight of the mixture.

3. Composition as claimed in claim 1 or 2, wherein the enzyme is capable of digesting down those types of dietary fibres which are more difficult to metabolize by the endogenous beneficial intestinal bacteria than other dietary fibres (xylanase).

4. Composition according to any one of the previous claims, whereby the nutrient is chosen from the group consisting of glutamine, vitamin A, vitamin C, vitamin D, vitamin E, vitamin B6, vitamin B9, Zinc and combinations thereof.

5. Composition according to any one of the previous claims, wherein the enzyme is chosen from the group consisting of xylanases, pectinases and combinations of two or more thereof.

6. Composition according to any one of the preceding claims, wherein additional enzymes are chosen from the group of lactase, amylase, amyloglucosidase, lipase and combinations of two or more thereof.

7. Composition according to any one of the previous claims, wherein the quantity of dietary fibres is 50-99 wt.%, preferably 70-95 wt.%, more preferably 80-95 wt.% with respect to the total weight of the composition.

8. Composition according to any one of the previous claims, wherein the quantity of glutamine is 2-10 wt.%, preferably 5-10 wt.%, with respect to the total weight of the composition.

9. Composition according to any one of the previous claims, wherein the quantity of enzymes is 1-5 wt.%, preferably 2-3 wt.% with respect to the total weight of the composition

10. Composition according to any one of the previous claims, wherein the quantity of vitamins and minerals is 0,1-5 wt%, preferably 1-3 wt.%, with respect to the total weight of the composition.

11. Composition according to any one of the previous claims, wherein the beneficial health effects are chosen from the group consisting of improvement of food digestion, treatment of diarrhoea, treatment of constipation, treatment of irritable bowel syndrome, treatment of inflammatory bowel disease, treatment of crohn's disease, treatment of obesity, treatment of dyslipidemia, and combinations of two or more thereof.

12. Composition according to any one of the previous claims for administration in a dose corresponding to 5-20 g per day, preferably 5-10 g per day.
